# EUROPEAN PATENT SPECIFICATION

(11) **EP 1 452 520 B1**
(45) Date of publication and mention of the grant of the patent: **25.05.2011**
(21) Application number: 03004131.3
(22) Date of filing: 26.02.2003
(51) Int. Cl.: C07C 215/24, C07C 247/08, C07D 413/04

(54) **A process for the synthesis of sphingosine**
Ein Verfahren zur Herstellung von Sphingosin
Procédé de préparation de la sphingosine

(43) Date of publication of application: 01.09.2004
(73) Proprietor: Cosmoferm B.V., 2600 MA Delft (NL)
(72) Inventor: Van Boom, Jacobus Hubertus, Prof. Dr., 2343 CR Oegstgeest (NL); Van den Berg, Richard, 2343 XZ Oegstgeest (NL)
(74) Representative: Lang, Arne

(56) References cited:
- VAN DEN BERG R J B H N ET AL: "A simple and low cost synthesis of d-erythro-sphingosine and d-erythro-azidosphingosine from d-ribo-phytosphingosine: glycosphingolipid precursors" TETRAHEDRON LETTERS, ELSEVIER SCIENCE PUBLISHERS, AMSTERDAM, NL, vol. 43, no. 46, 11 November 2002 (2002-11-11), pages 8409-8412, XP004388457 ISSN: 0040-4039
- SHIGEO SUGIYAMA ET AL.: "Stereochemistry of the Four Diastereomers of Ceramide and Ceramide Lactoside" LIEBIGS ANNALEN DER CHEMIE, 1991, pages 349-356, XP002248384 WEINHEIM DE
- RODNEY A FERNANDES ET AL: "A Stereoselective Synthesis of Dihydrosphingosine" EUROPEAN JOURNAL OF ORGANIC CHEMISTRY, WILEY-VCH VERLAG, WEINHEIM, DE, 2000, pages 3447-3449, XP002199352 ISSN: 1434-193X
- PÄIVI M KOSKINEN ET AL: "Sphingosine, an Enigmatic Lipid: A Review of Recent Literature Syntheses" SYNTHESIS, GEORG THIEME VERLAG. STUTTGART, DE, no. 8, 1998, pages 1075-1091, XP002199353 ISSN: 0039-7881

## Description

### BACKGROUND OF THE INVENTION.

Glycosphingolipids are important components of plasma membranes and play an important role in numerous biological processes, including cellular recognition processes and numerous signal transduction pathways. A vast number of naturally occurring glycosphingolipids are characterised by the presence of D-erythro-sphingosine [(2S,-3R,-4E)-2-amino-3-hydroxyoctadec-4-en-1-ol], the primary hydroxyl group of which is glycosylated while the amino function is acylated by a fatty acid, thus constituting a ceramide unit.

In the recent years, glycosphingolipids are gaining interest for cosmetic, pharmaceutical and therapeutic applications.

Although various lipids have been found to play a role in stratum corneum homeostasis, ceramides are assumed to be the main lipid components within the intercellular lipid membranes for holding the corneocytes together, and to be essential components in building and maintaining the barrier function of the stratum corneum. The observation that topical application of ceramides improves the barrier function of the stratum corneum as well as the moisture retaining properties of the skin, amongst others account for this increasing interest. Besides improving the function of the stratum corneum, topical application of ceramides has been found to assist in repairing damaged epidermal barrier function. Following these observations, a wide variety of cosmetic and pharmaceutical products containing sphingoid bases, ceramides and glycosphingolipids have been developed.

Initially, sphingolipids for cosmetic and pharmaceutical applications were obtained by extraction of animal tissue. However, the thus obtained sphingolipids are a heterogenous mixture of a plurality of structurally different sphingolipids, which moreover are potentially unsafe due to the possible presence of hazardous micro-organisms. Besides this, extraction from tissue is a laborious and expensive method, with a too low yield. In order to meet the growing demand for sphingolipids, numerous attempts have been made to develop synthetic pathways for producing sphingolipids and their sphingoid base building blocks.

A structural unit common to ceramide 1, 1a, 2, 4, 5 is D-*erythro-*sphingosine (formula 1 below). Examples of synthetic pathways to sphingosine are disclosed in Shapiro D. et al, J. Am. Chem. Soc. 1954, 76, 5897-5895, Lees W.J. Tetrahedron Letters, 2001, 42, 5845-5847, Compostella et al., Tetrahedron Letters, 2002, 58, 4425-4428.

In P.M. Koskinen, Synthesis 1998, 8, 1075-1091 several chemical synthetic routes for the synthesis of sphingosine are disclosed. One of the main issues in developing a suitable chemical process appears to be, the problem of obtaining sphingosine in its naturally active configuration, i.e. D-*erythro-*sphingosine. To achieve this, synthesis was started from chiral starting products, chirality was introduced in subsequent reaction steps through catalysis, or a chiral auxiliary was added in case a non-chiral compounds were used as starting compounds. However, all these chemical synthesis routes have in common that they involve a large number of reaction steps, with often a low yield, a low trans/cis selectivity of the olefinic bond and a low stereo specificity.

In R. Wil et al, Tetrahedron Asymmetry, 1994, 2195-2208, a method is disclosed for a stereoselective synthesis of D-erythro-sphingosine, starting from D-galactose. This method however comprises a large number of subsequent reaction steps, several of them giving incomplete conversion and a low selectivity to the desired product. In this chemical synthesis method, the key Grignard reaction for elongating 2,4-*O*-benzylidene-D-threose into the desired D-arabino configurated sphingosine precursor gave poor diastereo selectivity. Mesylation of this precursor favours the C-2 OH group, which is subsequently subjected to azide substitution to give a D-ribo phytosphingosine derivative. The 4,5-trans double bond is introduced by coupling a p-nitrobenzenesulfonyl moiety to the 4-hydroxyl group of the D-ribo-phytosphingosine derivative, and subjecting this coupled compound to a base induced elimination reaction, which favours the formation of the 4,5 sphingosine double bond. Two further reaction steps finally gave the desired D-*erythro*-sphingosine.

Phytosphingosine, another sphingoid base, in contrast to sphingosine is commercially available on an industrial scale. A biotechnological process for the production pf phytosphingosine by mutant yeast strains is disclosed in EP-A-726.960.

Richard et al. in A simple and low cost synthesis of D-erythro-sphingosine and D-erythro-azidosphingosine from D-ribo-phytosphingosine: glycosphingolipid precursors, Tetrahedron Letters, Volume 43, Issue 46, 2002, Pages 8409-8412, disclose a method for preparation of D-erythro-Sphingosine and D-erythro-2-azidosphingosine from D-ribo-phytosphingosine by palladium catalyzed regiospecific reduction of a Z-enol triflate generated by different steps of silylation, oxidation and sulfonylation of the D-ribo-phytosphingosine.

### SUMMARY OF THE INVENTION.

There is thus a need to have sphingolipids, in particular sphingoid bases such as sphingosine and phytosphingosine as well as the ceramides based on these compounds, available in commercially interesting amounts against a reasonable cost. Therefore, it is an object of this invention to provide an economically feasible process for the production of sphingoid bases, in particular a process for the production of sphingosine, more particularly D-*erythro*-sphingosine (1) starting from phytosphingosine (2), to be used on an industrial scale.

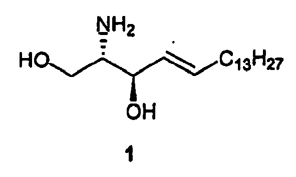

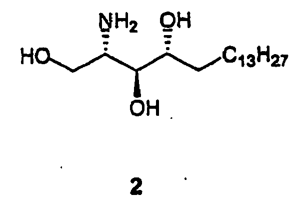

Sphingoid bases that are compatible with human tissue in general correspond to the formula below: 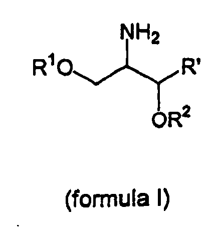 in which
R' is a hydrocarbon chain having 7-52, preferably 15-21 carbon atoms, which may be a straight chain or branched, which may be saturated or contain one or more double bonds, which may contain one or more functional groups, the functional groups being preferably selected from the group of a hydroxyl group, an alkoxy group, a primary, secondary or tertiary amine, a thiol group, a thioether or a phosphorous containing functional group, a ketone, an ester residue. Preferably however, the functional group is a OH-group. In case R' is branched, the side chains preferably contain 1-4 carbon atoms, more preferably comprise a methyl group.
R¹ and R² may be the same or different and may be H or a hydrocarbon chain having 1-10, preferably 1-5 carbon atoms, which may be a straight chain or branched, which may be saturated or contain one or more double bonds, which may contain one or more functional groups. Preferably however, R¹ and R² are H.

The present invention concerns a process for the production of a sphingoid base, according to formula 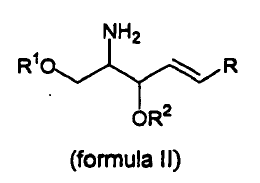 in which
R is a hydrocarbon chain having 5-50, preferably 13-19 carbon atoms, which may be a straight chain or branched, which may be saturated or contain one or more double bonds, which may contain one or more functional groups, the functional groups being preferably selected from the group of a hydroxyl group, an alkoxy group, a primary, secondary or tertiary amine, a thiol group, a thioether or a phosphorous containing functional group, a ketone, an ester residue,
R¹ and R² are H.

An economically feasible process for the production of the sphingoid base of formula II may be obtained by using as a starting compound the organic base of formula III below and subjecting this compound to a process comprising the steps of:
(1) dissolving a starting compound according to formula III or a salt thereof in a substantially inert solvent, 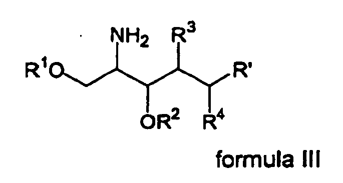 in which R¹, R², are as defined above
   R³ and R⁴ may be the same or different and may be H, OH, or a hydrocarbon chain having 1-10, preferably 1-5 carbon atoms, which may be a straight chain or branched, which may be saturated or contain one or more double bonds, which may contain one or more functional groups,
   and R' is a hydrocarbon chain having 3-48, preferably 11-17 carbon atoms, which may be a straight chain or branched, which may be saturated or contain one or more double bonds, which may contain one or more functional groups, the functional groups being preferably selected from the group of a hydroxyl group, an alkoxy group, a primary, secondary or tertiary amine, a thiol group, a thioether or a phosphorous containing functional group, a ketone, an ester residue,
(2) protecting the NH₂ group with a NH₂ protecting group,
(3) activating the C₄HR³ for an elimination reaction with C₅HR⁴,
(4) causing an elimination reaction to take place to form a double bond between the C₄ and C₅ carbon atoms,
(5) Removing the NH₂ protecting group. This may be done immediately as part of the process or later on after having stored the compound for some period.

In the process of this invention, care is taken to first protect the NH₂ function with a protecting group and to carry out the activation of the C₄HR³⁻ for the elimination reaction with C₅HR⁴ only thereafter. This is done to allow separately controlling the reactions occurring on both the C₄HR³⁻ and NH₂ sites.

The starting compound used in the method of this invention is a sphingoid base corresponding to formula (V):

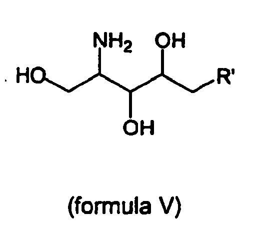

In which R' is as defined above.

The preferred compounds of formula V are those having the D-ribo configuration, as this is the stereochemical configuration, which is most abundant in the stratum corneum. The compounds of formula (V) may however also take the D-threo configuration or any other configuration known to the man skilled in the art.

More preferably as a starting compound use is made of sphinganine, phytosphingosine, monoacetylphytosphingosine, tetra-acetylphytosphingosine, monoacylphytosphingosine or a derivative or a salt thereof, phytosphingosine as such being the particularly preferred starting product as this is readily commercially available.

When using phytosphingosine or a derivative or a salt thereof as a starting compound, the process of the invention comprises the steps of:
(1) protecting the NH₂ group with a NH₂ protecting group,
(2) thereafter protecting the C₁OH group with a protecting group selective to the C₁OH group
(3) and protecting the C₃OH group with a protecting group selective to the C₃OH group
(4) activating the C₄ OH for an elimination reaction with C₅H,
(5) causing an elimination reaction to take place to form a double bond between the C₄ and C₅ carbon atoms,
(6) removing the OH protecting groups followed by removing the NH₂ protecting group.

When using sphinganine or a derivative or a salt thereof as a starting compound, the process of the invention comprises the steps of:
(1) protecting the NH₂ group with a NH₂ protecting group,
(2) thereafter protecting the C₁OH group with a protecting group selective to the C₁OH group
(3) oxidizing the C₃OH to a C=O,
(4) rearranging the C=O to the corresponding allylic keton,
(5) reduction of the keton function of the allylic keton into an allylic alcohol,
(6) removing the OH protecting groups followed by removing the NH₂ protecting group.

Protection of the NH₂ group is achieved in the described below:

The compound of formula IV is caused to react with an organic acid, an activated ester, an acid halogenide, an acid anhydride, to convert the amine function to the corresponding amide. Particularly preferred is the acid halogenide according to formula XI

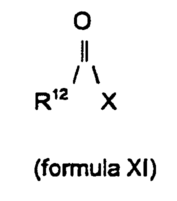

In which R¹² may be a hydrocarbon group having 1-50 carbon atoms, which may be a straight chain or branched, saturated or contain one or more unsaturated bonds, cyclic, armomatic, contain functional groups for example OH, SH etc. The advantage is that the risk to inversion of the optical activity at the C-2 may be minimised, this compound showing a high selectivity towards reaction with the NH₂ group.

In first instance the acid halogenide will react with the NH₂ group.

A preferred acid halogenide is benzoyl chloride or acetyl chloride as this allows formation of a stable amide bond.

For the protection of the C-1 OH group this group is activated by a sulfonyl reagent allowing an intramolecular reaction with the amide bond to form a five memebered 2-substituted oxazoline. For example for benzoylamide this will give the 2-phenyl-oxazoline and the acetylamide will give 2-methyl-oxazoline.

When proceeding with the reaction it is important to ensure that the risk to inversion of the optical activity at the C-3 is also minimised. In particular it should be ensured to counteract reaction between the NH₂-benzoyl group and the C₃OH and the C₄OH, which is favourable as it gives rise to the formation of a 5 membered ring, as such reaction would involve inversion of the optical activity at the C-3, or rendering the C₄OH less suitable for the desired elimination reaction. This object may be achieved by a careful selection of the protecting groups for the C₃OH and C₄OH functional groups.

After having coupled the NH₂ and C₁OH group to the protective group as a 2-substituted oxazoline, the C₉OH and C₄OH are converted into an epoxide. This may be done trough epoxidation by first sulfonylating the OH group followed by application of alkaline conditions or by converting the C₃OH and C₄OH to an ortho ester, for example by causing it to react with trimethylorthoformate, followed by a substitution with TMSX or AcX in which X is a halogen and applying strong alkaline conditions to induce saponication of the formed ester followed by the subsequent epoxide formation.

This may also be achieved by first converting the C₄OH group into a good leaving group as has been described above and by converting the leaving group into an epoxide in the presence of a strong base, in particular a sterically hindered base so as to avoid nucleophilic substitution reactions.

Formation of the epoxide has the advantage that inversion of the optical activity at the C-3 carbon atom may be minimised, by making a particular choice of ring opening reactants. This may be achieved by causing the epoxide to react with TMSX in which X is a halogen or triflate, preferable iodine followed by strong base treatment involving the simultaneous formation of the (sphingosine) double bond between the C-4 and ―C-5 carbon atoms. Surprisingly, leaving of the halogene or sulfonyl group appears to be associated with an β-elimination reaction, involving the simultaneous formation of the (sphingosine) double bond between the C-4 and -C-5 carbon atoms. The elimination reaction appears to take place with virtually complete selectivity towards the desired end product, which appeared to be virtually free of unwanted side products.

Subsequent protic acid treatment followed by alkaline treatment give D-*erytho*-sphingosine as a pure and sole product.

The invention is further illustrated in the following examples.

### Analytical methods.

### General methods and materials.

¹H NMR and ¹³C NMR spectra were recorded with a Bruker WM-200 (200/50.1 MHz), Bruker WM-300S (300/75.1 MHz) spectrometer. ¹H and ¹³C chemical shifts are given in ppm (δ) relative to tetramethylsilane (δ=0.00), CDCl₃ (δ=77.00) as internal standard. Electrospray mass-spectra were recorded using a Perkin-Elmer SCIEX API 165 Single Quadruple LC/MS instrument. Dichloromethane, pyridine, tetrahydrofuran, toluene was dried by molecular sieves (4A). Column chromatography was performed on Silica gel 60 (220-440 mesh ASTM, Fluka). TLC-analysis was performed with silica gel TLC-plates (Merck, Silica gel, F₂₅₄) with detection by UV-absorption (254 nm) where applicable and charring (±140°C) with 20% H₂SO₄ in MeOH or ammonium molybdate (25 g/L) and ceric ammonium sulfate (10 g/L) in 20% H₂SO₄. Compounds containing NH₂-functions were visualized by spraying TLC-plates with a solution of ninhydrin (0.30 g) in *n*-butanol (100 mL) containing acetic acid (3 mL) followed by charring ±140°C. Prior to reactions that require anhydrous conditions, traces of water were removed by coevaporation with dry toluene. These reactions were conducted under dry argon atmosphere.

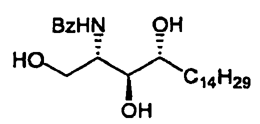

### Example 1 Preparation of (2S,3S,4R)-2-N-Benzoylamino-1.3.4-octadecanetriol.

To a stirred suspension of phytosphingosine (10.0 g, 31.5 mmol) in THF (250 mL) containing TEA (5.27 ml, 37.8 mmol) was added benzoylchloride (3.84 mL, 33.1 mmol) and stirred for 30 min. The solvent was evaporated and the residue dissolved in hot ethyl acetate (250 mL), washed with aqueous HCl (1 N, 2 × 50 mL), after cooling the organic layer to 0°C white crystals were obtained. Yield 13.1 g (99%).

TLC (silica gel, diethylether/ethanol/ammoniumhydroxide, 6:3:1): *R_{f}* = 0.75. mp 114°C.

ES-MS: *m*/*z* 422.3 [M + H]⁺, 444.6 [M + Na]⁺, 865.9 [2M + Na]⁺.

Optical rotation 13.6° (*c*= 1.07 CHCl₃/MeOH, 5:1, v/v).

¹H-NMR (CD₃0D/CDCl₃): δ = 0.88 (t, 3H, CH₃), 1.28 (s, 24H, 12 × CH₂), 1.49 (br. s, 2H, CH₂), 3.63 (m, 1H, H-4), 3.70 (m, 1H, H-3), 3.89 (m, 2H, H₂-1), 4.34 (m, 1H, H-2), 4.49 (s, 3H, 3 × OH), 7.41-7.90 (m, 5H, CH-arom benzoyl).

¹³C{¹H}-NMR (CDCl₃): δ = 13.4 (CH₃), 22.2, 25.3, 28.9, 29.2, 31.5, 32.6 (× CH₂), 52.2 (C-2), 60.7 (C-1), 72.1, 75.1 (C-3 / C-4), 126.7, 128.1, 131.3 (CH-arom benzoyl), 133.1 (Cq benzoyl), 168.3 (C=O benzoyl).

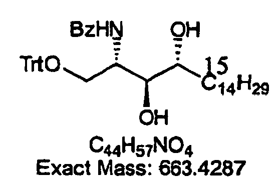

### Example 2. Preparation, of (2S.3S.4R)-2-N-Benzoylamino-1-O-trityl-1,3,4-octadecanetriol.

To a stirred suspension of (2S,3S,4R)-2-*N-*benzoylamino-1,3,4-octadecanetriol (58.9 g, 140 mmol) in EtOAc (600 mL) containing TEA (23 ml, 138 mmol), maintained at 80°C was dropwise added a solution of triphenylmethyl chloride (42.9 g, 138 mmol) in EtOAc (200 mL). The mixture was stirred at 80°C for 2.5 h. The solvent was washed successively with aqueous HCl (1N, 3 × 200 mL), aqueous NaHCO₃ (10%, 1 × 200 mL) and concentrated. Column chromatography of the residue over silica gel with toluene/EtOAc (1:0 → 3:2, v/v) gave title compound (92.8 g, 99%) as a colorless oil. *R_{f}* = 0.80 (MeOH/EtOAc, 1:9).

ES-MS: *m*/*z* 686.7 [M + Na]⁺.

Optical rotation 11.2° (*c* =1.04 CHCl₃).

¹H-NMR (CD₃OD/CDCl₃): δ = 0.87 (t, 3H, CH₃), 1.25 (s, 24H, 12 × CH₂), 1.44 (m, 2H, CH₂), 2.57 (br. t, 1H, OH-3), 3.35 (dd, 1H, H-3, *J*_{2,3} = 8.0 Hz, *J*_{3,4} = 8.4 Hz), 3.47 (dd, 2H, H-1a, OH-4, *J*_{1a,1b} = 9.9 Hz, *J*_{1a,2} = 4.4 Hz), 3.60 (dd, 1H, H-1b, *J*_{1a,1b} = 9.9 Hz, *J*_{1a,2} = 3.3 Hz), 3.69 (m, 1H, H-4), 4.47 (m, 1H, H-2, *J*_{2,NH} = *J*_{2,3} = 8.0 Hz, *J*_{1a,2} = 4.4 Hz, *J*_{1b,2} = 3.3 Hz), 6.90 (d, 1H, NH, *J*_{2,NH} = 8.0 Hz), 7.16 - 7.75 (m, 20H, CH-arom benzoyl/Trt).

¹³C{¹H}-NMR (CDCl₃): δ = 14.0 (CH₃), 22.5, 25.6, 29.2, 29.5, 31.7 (× CH₂), 50.9 (C-2), 62.9 (C-1), 72.8, 75.5 (C-3 / C-4), 87.3 (Cq Trt), 126.9 - 131.4 (CH-arom benzoyl/Trt), 134.0 (Cq benzoyl), 143.1 (Cq Trtarom), 167.2 (C=O benzoyl).

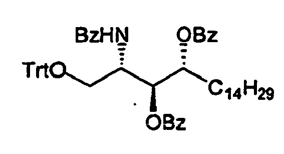

### Example 3. Preparation of (2S,3S,4R)-2-N-Benzoylamino.3,4-O-dibenzoyl-1-O-trityl-1,3,4-octadecanetriol.

To a stirred solution of (2S,3S,4R)-2-*N*-benzoylamino-1-*O-*trityl-1,3,4-octadecanetriol (17.5 g, 26 mmol) in EtOAc (50 mL) containing TEA (8.0 mL, 57 mmol) was dropwise added benzoyl chloride (6.6 mL, 57 mmol) and stirred overnight. Excess of benzoyl chloride was quenched by the addition MeOH (5 mL). The mixture was diluted with EtOAc (50 mL) and successively washed with aqueous HCl (1N, 3 × 100 mL), aqueous NaHCO₃ (10%, 2 × 50 mL), dried (MgSO₄), and concentrated. Column chromatography of the residue over silica.gel with petroleum ether/EtOAc (95:5 → 80:20, v/v) gave title compound (24.0 g, 100%) as a colorless oil. *R_{f}* = 0.90 (petroleum ether/EtOAc, 2:1).

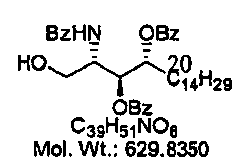

### Example 4. Preparation of (2S,3S,4R)-2-N-Benzoylamino-3.4-O-dibenzoyl-1,3,4-octadecanetriol.

To a solution of (2S,3S,4R)-2-*N*-benzoylamino-3,4-*O-*dibenzoyl-1-*O-*trityl-1,3,4-octadecanetriol (52.4 g, 60.2 mmol) in MeOH/toluene (400 mL, 1:1, v/v) was added BF₃·O(Et)₂ (11.3 mL, 90.3 mmol) and the mixture was stirred for 1.5 h. The mixture was diluted with EtOAc (100 mL) and washed with aqueous NaHCO₃ (10%, 3 × 100 mL), dried (MgSO₄) and concentrated. Column chromatography of the residue over silica gel with petroleum ether/EtOAc (95:5 → 33:66, v/v) gave title compound (94 g, 99%) as a yellow oil. TLC (silica gel, petroleum ether/EtOAc, 2:1): *R_{f}* = 0.40.

Optical rotation 50.6° (c = 1.14 CHCl₃).

ES-MS: *m*/*z* 630.5 [M + H]⁺, 652.4 [M + Na]⁺.

¹H-NMR (CDCl₃): δ = 0.87 (t, 3H, CH₃), 1.21 (s, 24H, 12 × CH₂), 2.06 (br. s, 2H, CH₂), 3.00 (t, 1H, OH), 3.75 (m, 2H, CH₂-1), 4.60 (m, 1H, H-2), 5.47 (m, 1 H, H-4), 5.60 (dd, 1H, H-3), 7.21 (d, 1 H, NH), 7.33 - 8.08 (m, 15H, CH-arom benzoyl).

¹³C{¹H}-NMR (CDCl₃): δ = 14.0 (CH₃), 22.6, 25.6, 28.3, 29.5, 31.8, (x CH₂), 50.7 (C-2), 61.3 (C-1), 73.1, 74.0 (C-3 / C-4), 127.1 - 133.9 (CH-arom benzoyl), 166.5, 167.5, (C=O benzoyl).

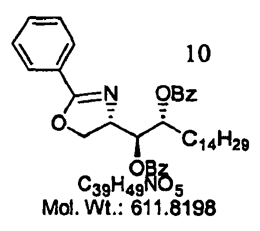

### Example 5. Preparation of 1-[4-(S)-(2-Phenyl-4,5-dihydro-oxazol-4-yl)]-(1S,2R)-hexadecane-1.2-O-dibenzoyl.

To solution of (2S,3S,4R)-2-*N*-benzoylamino-3,4-*O-*dibenzoyl-1,3,4-octadecanetriol (32.0 g, 50.8 mmol) in DCM (250 mL) containing TEA (75 mL, 531 mmol), maintained at 0°C was added methanesulfonyl chloride (8.22 mL, 106.2 mmol). After 1 h, the reaction mixture was allowed to raise to ambient temperature and stirred for 18 h. The reaction was washed with aqueous HCl (1N. 3 × 200 mL), aqueous NaHCO₃ (10%, 200 mL), dried (MgSO₄) and concentrated. The residue was purified by silica gel column chromatography. Elution was performed with petroleum ether/EtOAc (95:5 → 85:15, v/v) giving 26.7 g (86%) of colorless oil. *R_{f}* = 0.80 (petroleum ether/EtOAc, 3:1, v/v).

Optical rotation ―50.0° (*c* = 1.42 CHCl₃).

ES-MS: *m*/*z* 612.5 [M + H]⁺, 634.7 [M + Na]⁺.

¹H-NMR (CDCl₃): δ = 0.88 (t, 3H, CH₃), 1.24 (s, 24H, 12 × CH₂), 1.91 (dd, 2H, CH₂), 4.53 (dd, 1H, H-1, *J*_{1,1'} = 8.8 Hz, *J*_{1,2} = 10.2 Hz), 4.62 (dd, 1H, H-1', *J*_{1,1'} = 8.4 Hz, *J*_{1',2} = 6.9 Hz), 4.77 (ddd, 1H, H-2, *J*_{1,2} = 10.2 Hz, *J*_{1',2} = 6.6 Hz, *J*_{2,3} = 3.7 Hz), 5.62 (m, 2H, H-3, H-3), 7.26 - 8.04 (m, 15H, CH-arom benzoyl, phenyloxazol).

¹³C{¹H}-NMR (CDCl₃): δ = 14.0 (CH₃), 22.6, 25.3, 29.3, 29.6, 30.6, 31.8 (x CH₂), 66.6 (C-2), 69.1 (C-1), 73.4, 75.4 (C-3 / C-4), 127.2 - 133.1 (CH-arom benzoyl, phenyloxazol), 165.3, 165.5, 165.7, (C=N, C=O benzoyl, phenyloxazol).

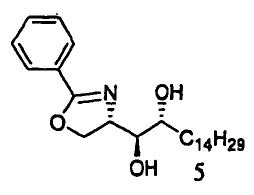

### Example 6. Preparation of 1-[4-(S)-(2-Phenyl-4,5-dihydro-oxazol-4-yl)]-(1S,2R)-hexadecane-1,2-diol.

Potassium carbonate (15.0 g, 108 mmol) was added to a mixture of 1-(4-(*S*)-(2-phenyl-4,5-dihydro-oxazol-4-yl)]-(1*S*,2*R*)-hexadecane-1,2-*O-*dibenzoyl (13.4 g, 21.8 mmol) in chloroform/MeOH (100 mL, 3:1, v/v) and stirred for 18 h at 60°C. TLC analysis indicated the complete conversion of starting material into a compound with *R_{f}* = 0.60 (DCM/MeOH, 9:1, v/v). After evaporating the solvents, the residue was dissolved in hot EtOAc (500 mL) washed with water (3 × 100 mL) and the organic layer concentrated. The crystals were crystallized from hot petane yielding 8.8 g of white crystals (100%).

mp 137 - 138°C.

Optical rotation 24.8° (c = 1.60 CHCl₃/methanol, 5:1, v/v).

ES-MS: *m*/*z* 404.3 [M + H]⁺, 426.1 [M + Na]⁺, 4.44.5 [M + K]⁺, 807.6 [2M + H]⁺, 829.5 [2M + Na]⁺, 847.9 [2M + K]⁺.

¹H-NMR (CDCl₃): δ = 0.88 (t, 3H, CH₃), 1.25 (s, 24H, 12 × CH₂), 1.52 (m, 2H, CH₂), 2.17 (d, 1H, OH), 3.27 (d, 1H, OH), 3.71 (m, 1H, H-3), 3.81 (m, 1H, H-4), 4.40 - 4.61 (m, 3H, H₂-1, H-2), 7.26 - 7.92 (m, 5H, CH-arom phenyloxazol). ¹³C{¹H}-NMR (CDCl₃): δ = 13.9 (CH₃), 22.5, 25.4, 29.1, 29.5, 31.7, 32.9 (x CH₂), 68.4 (C-2), 69.3 (C-1), 73.5, 74.9 (C-3 / C-4), 126.9 - 132.9 (CH-arom phenyloxazol), 165.4 (C=N phenyloxazol).

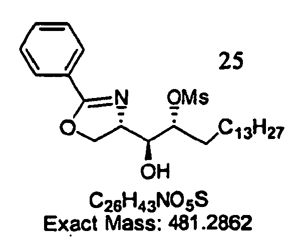

### Example 7. Preparation of 1-[4-(S)-(2-Phenyl-4,5-dihydro-oxazol-4-yl)]-(1S,2R)-1-hvdroxy-2-O-mesyl-hexadecane.

To a solution of 1-[4-(*S*)-(2-phenyl-4,5-dihydro-oxazol-4-yl)]-(1*S*,2*R*)-hexadecane-1,2-diol (1.148 g, 2.84 mmol) in chloroform/pyridine (20 mL, 1:1, v/v) was added methane sulfonyl chloride (0.231 mL, 2.98 mmol) and stirred for 8 h. Methanol (0.5 mL) was added and the solvent was evaporated in vacuo. The residue was taken up in EtOAc (50 mL) and washed successively with aqueous HCl (1N, 3 × 50 mL), aqueous NaHCO₃ (10%, 50 mL), brine (50 mL), dried (MgSO₄) and concentrated. Column chromatography if the residue over silica gel with petroleum ether/EtOAc (9:1 → 7:3, v/v) gave title coumpound (0.917 g, 67%) as a colorless oil. TLC (silica gel, petroleum ether/EtOAc, 2:1): *R_{f}* = 0.50.

¹H-NMR (CDCl₃): δ = 0.88 (t, 3H, CH₃), 1.26 (s, 24H, 12 × CH₂), 1.82 (m, 2H, CH₂), 3.12 (s, 3H, Ms), 4.19 (d, 1H, H-3), 4.47 (m, 3H, H₂-1, H-2), 4.86 (dt, 1H, H-4), 7.27 - 7.74 (m, 5H, CH-arom phenyloxazol).

¹³C{¹H}-NMR (CDCl₃): δ = 14.0 (CH₃), 22.5, 24.9, 29.5, 31.3, 31.7, (x CH₂), 38.3 (CH₃ Ms), 67.1 (C-2), 67.7 (C-1), 71.6, (C-3), 84.2 (C-4), 126.4 (Cq phenyloxazol), 128.0, 128.1, 131.4 (CH-arom phenyloxazol), 165.7 (C=N phenyloxazol).

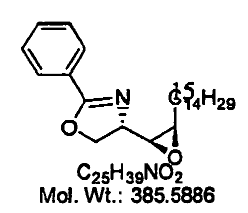

### Example 8. Preparation of 1-[4-(S)-(2-Phenyl-(4,5-dihydro-oxazol)-4-yl]-(1S,2R)-1,2-oxiranyl-hexadecane.

To a solution of 1-[4-(*S*)-(2-Phenyl-4,5-dihydro-oxazol-4-yl)]-(1*S*,2*R*)-1-hydroxy-2-*O-*mesyl-hexadecane (0.208 g, 0.43 mmol) in THF (4 mL), maintained at 0°C, was added potassium *tert*-butylate (0.048 g, 0.43 mmol), and the mixture was stirred for 2 h. The mixture was diluted with EtOAc (50 mL) and successively washed with water (2 × 20 mL), brine (20 mL), dried (MgSO₄) and concentrated. Column chromatography of the residue over silica gel with petroleum ether/EtOAc EtOAc (x:x → x:x, v/v) gave title compound (0.166 g, 99%) as a crystalline solid. TLC (silica gel, petroleum ether/EtOAc, 1:1): *R_{f}* = 0.70.

Optical rotation 24.8° (c = 1.60 CHCl₃/MeOH 5:1, v/v).

ES-MS: *m*/*z* 386.3 [M + H]⁺, 408.2 [M + Na]⁺.

¹H-NMR (CDCl₃): δ = 0.88 (t, 3H, CH₃), 1.25 (s, 24H, 12 × CH₂), 1.55 (m, 2H, CH₂), 2.95 (dd, 1H, H-4), 3.05 (dt, 1H, H-3), 4.27 (m, 1H, H-2), 4.41 - 4.56 (m, 2H, H₂-1), 7.35 - 7.97 (m, 5H, CH-arom phenyloxazol).

¹³C{¹H}-NMR (CDCl₃): δ = 14.0 (CH₃), 22.7, 25.9, 29.4, 29.6, 31.6, 33.4 (x CH₂), 55.8, 59.1 (C-3 / C-4), 66.4 (C-2), 68.9 (C-1), 128.3 - 131.5 (CH-arom phenyloxazol), 165.2 (C=N phenyloxazol).

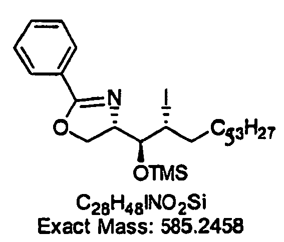

### Example 9. Preparation of 1-[4-(S)-(2-Phenyl-4,5-dihydro-oxazol)-4-yl]-(1S,2R)-2-iodo-1-trimethylsilanyloxyhexadecane.

To a solution of 1-[4-(*S*)-(2-Phenyl-(4,5-dihydro-oxazol)-4-yl]-(1*S*,2*R*)-1,2-oxiranyl-hexadecane (0.157 g, 0.41 mmol) in acetornitrile (4 mL), maintained at 40°C, were added DBN (0.097 mL, 0.82 mmol) and TMSI (0.061 mL, 0.45 mmol) and the mixture was stirred for 3 h. The solvent was evaporated and the residue taken up in EtOAc (50 mL) and washed successively with aqueous HCl (1N, 2 × 50 mL), aqueous NaHCO₃ (10%, 50 mL), brine (50 mL), dried (MgSO₄) and concentrated. Column chromatography of the residue over silica gel with petroleum ether/EtOAc (10:0 → 9:1, v/v) gave title compound (0.14 g, 59%) as a crystalline solid. TLC (silica gel, petroleum ether/EtOAc, 1:1): *R_{f}* = 0.90.

ES-MS: *m*/*z* 386.3 [M + H]⁺, 408.2 [M + Na]⁺.

¹H-NMR (CDCl₃): δ = 0.08 (s, 9H, TMS), 0.88 (t, 3H, CH₃), 1.26 (s, 22H, 11 × CH₂), 1.63 (m, 2H, CH₂), 1.82 (m, 2H, CH₂), 4.01 - 4.15 (m, 2H, H-3, H-4), 4.35 - 4.49 (m, 2H, H₂-1), 4.80 (ddd, 1H, H-2), 7.36 - 7.96 (m, 5H, CH-arom phenyloxazol). ¹³C{'H}-NMR (CDCl₃): δ = 0.6 (CH₃ TMS), 14.1 (CH₃), 22.7, 28.8, 29.6, 31.9, 35.4 (x CH₂), 41.0 (C-4), 68.0 (C-1), 70.6 (C-2), 78.2 (C-3), 127.7 (Cq phenyloxazol), 128.2, 128.3, 131.3 (CH-arom phenyloxazol), 164.3 (C=N phenyloxazol).

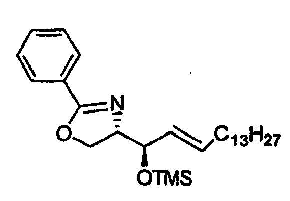

### Example 10. Preparation of 1-[4-(S)-(2-Phenyl-4,5-dihydro-oxazol)-4-yl]-(1S,2E)-1-trimethylsilanyloxyhexadec-2-enyl.

To a solution of 1-[4-(*S*)-(2-Phenyl-(4,5-dihydro-oxazol)-4-yl]-(1*S*,2*R*)-1,2-oxiranyl-hexadecane ((0.456, 1.18 mmol) in acetonitrile (10 mL), maintained at 40°C was added TMSI (0.177 mL, 1.30 mmol) and the mixture was stirred for 20 min at 40°C. DBN (0.465 mL, 3.89 mmol) was added and the mixture was refluxed for 1.5 h. Subsequenity, the solvent was concentrated and column chromatografy of the residue using petroleum ether/EtOAc (1:0 → 4:1) gave title compound (0.375, 69%) as a colorless oil. TLC (silica gel, petroleum ether/EtOAc, 4:1): *R_{f}* = 0.90.

Optical rotation 6.2° (*c* = 1.28 CHCl₃).

ES-MS: *m*/*z* 458.4 [M + H]⁺, 480.4 [M + Na]⁺.

¹H-NMR (CDCl₃): δ = 0.04 (s, 9H, TMS), 0.87 (t, 3H, CH₃), 1.26 (s, 22H, 11 × CH₂), 1.37 (br. t, 2H, CH₂), 2.05 (dd, 2H, CH₂), 4.27 (dd, H-1 a), 4.28 (m, 1H, H-2), 4.39 (m, 1H, H-3), 4.42 (dd, 1H, H-1b), 5.45 (ddt, 1H, H-4, *J*_{4,5} = 15.2 Hz, *J*_{3,4} = 6.6 Hz), 5.71 (ddt, 1H, H-5, *J*_{4,5} = 15.2 Hz, *J*_{5,6} = 6.9 Hz), 7.37 - 7.95 (m, 5H, CH-arom phenyloxazol).

¹³C{¹H}-NMR (CDCl₃): δ = 0.3 (CH₃ TMS), 14.1 (CH₃), 22.6, 29.1, 29.2, 29.3, 29.4, 29.6, 31.9, 32.2 (× CH₂), 67.9 (C-1), 71.7 (C-2), 73.8 (C-3), 127.9 (Cq phenyloxazol), 128.2, 131.1 (CH-arom phenyloxazol), 129.9 (C-4), 132.1 (C-5), 164.4 (C=N phenyloxazol).

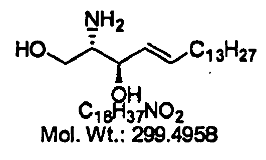

### Example 11. Preparation of (2S,3S,4E)-2-Amino-octadec-4-ene-1,3-diol (D-erythro-sphingosine).

To a solution of 1-[4-(*S*)-(2-Phenyl-4,5-dihydro-oxazol)-4-yl]-(1*S*,2*E*)-1-trimethylsilanyloxyhexadec-2-enyl (0.133, 0.29 mmol) in THF (5 mL) was added aqueous HCl (2 N, 4.8 mL), and stirred for 18 h. The mixture was diluted with chloroform/MeOH (20 mL, 87:13 v/v) and water (10 mL). The organic phase was separated and the aqueous phase extracted with a chloroform/methanol mixture (2 × 20 mL, 87:13 v/v). The combined organic layers were dried (MgSO₄) and concentrated. The residue was dissolved in MeOH (5 mL) and aqueous NaOH (50 eq), was added and the mixture refluxed for 2 h. The cooled reaction mixture was diluted with water (10 mL) and extracted with diethyl ether (3 × 20 mL). The organic layer was dried (MgSO₄) and concentrated. Column chromatography of the residue over silica gel with EtOAc/MeOH/NH₄OH (90:10:0 → 40:10:1) gave title compound (0.073g, 85%) as a white solid. TLC (silica gel, diethyl ether/EtOH/NH₄OH, 6:3:1): *R_{f}* = 0.50.

Optical rotation -1.4° (*c* = 0.42 CHCl₃).

ES-MS: *m*/*z* 300.4 [M + H]⁺, 322.4 [M + Na]⁺.

¹H-NMR (CDCl₃): δ = 0.87 (t, 3H, CH₃), 1.24 (s, 22H, 11 × CH₂), 2.03 (q, 2H, CH₂), 2.87 (br. s, 5H, H-2, NH₂, 2 × OH), 3.65 (br. s, 2H, H₂-1), 4.06 (br. s, 1H, H-3), 5.44 (dd, 1 H, H-4, *J*_{4,5} = 15.4 Hz, *J*_{3,4} = 5.9 Hz), 5.71 (dt, 1H, H-5, *J*_{4,5} = 15.4 Hz, *J*_{5,6} = 6.6 Hz).

Hertweck, C.; 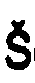 ebek, P.; Svato̅s

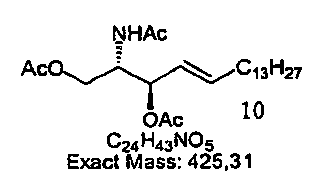

### Example 12. Preparation of (2S,3S,4E)-2-Acetamido-1,3-diacetvl-octadec-4-ene-1.3-diol.

To a solution of 1-[4-(*S*)-(2-Phenyl-4,5-dihydro-oxazol)-4-yl]-(1*S*,2*E*)-1-trimethylsilanyloxyhexadec-2-enyl (0.322, 0.70 mmol) in THF (x mL) was added

aqueous HCl (2 N, x.x mL), and stirred for 18 h. The mixture was diluted with chloroform/MeOH (20 mL, 87:13 v/v) and water (10 mL). The organic phase was separated and the aqueous phase extracted with a chloroform/methanol mixture (2 × 20 mL, 87:13 v/v). The combined organic layers were dried (MgSO₄) and concentrated. The residue was dissolved in MeOH (xx mL) and aqueous NaOH (50 eq) and refluxed for 2 h. The cooled mixture was diluted with water (10 mL) and extracted with diethyl ether (3 × 20 mL). The organic layer was dried (MgSO₄) and concentrated. The residue was dissolved in pyridine (2 mL) and acetic anhydride (2 mL) was added, and the mixture was stirred for 4 h. The mixture was concentrated. Column chromatography of the residue over silica gel with petroleum ether/EtOAc (50:50 → 0:100) gave title compound (0.240 g, 80%) as a white solid. TLC (silica gel, petroleum ether/EtOAc, 1:1): *R_{f}* = 0.20.

Optical rotation -13.2° (c = 1.00 CHCl₃).

¹H-NMR (CDCl₃): δ =

¹³C{¹H)-NMR (CDCl₃): δ =

## Claims

1. A method for the production of a sphingoid base
according to formula 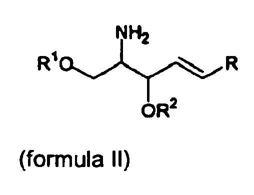 in which
R is a hydrocarbon chain having 5-50, preferably 13-19 carbon atoms, which may be a straight chain or branched, which may be saturated or contain one or more double bonds, which may contain one or more functional groups,
R¹ and R² are H,
this method comprising the steps of
(1) dissolving a starting compound according to formula III or a salt thereof in a
substantially inert solvent, 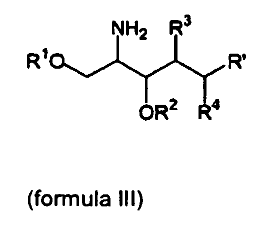 in which R¹, R², are as defined above
R³ and R⁴ may be the same or different and may be H, OH, or a hydrocarbon chain having 1-10, preferably 1-5 carbon atoms, which may be a straight chain or branched, which may be saturated or contain one or more double bonds, which may contain one or more functional groups,
and R' is a hydrocarbon chain having 3-48, preferably 11-17 carbon atoms, which may be a straight chain or branched, which may be saturated or contain one or more double bonds, which may contain one or more functional groups, the functional groups being preferably selected from the group of a hydroxyl group, an alkoxy group, a primary, secondary or tertiary amine, a thiol group, a thioether or a phosphorous containing functional group,
wherein as the compound of formula III use is made of a compound corresponding to formula V: 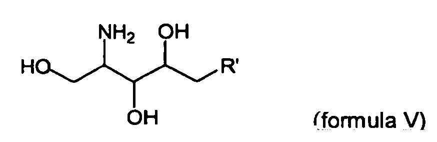 in which R' is as defined above
(2) protecting the NH₂ group with a NH₂ protecting group and causing the compound of formula III to react with an organic acid, an activated ester, an acid halogenide, an acid anhydride, to convert the amine function to the corresponding amide,
(3) activating C₄HR³ for an elimination reaction with C₅HR⁴,
(4) causing an elimination reaction to take place to form a double bond between the C₄ and C₅ carbon atom,
(5) removing the NH₂ protecting group,
**characterised in that** in the obtained amide in step (2) the NH group is caused to react with the C₁OH group to form the corresponding oxazoline compound and that the C₃OH and C₄OH are converted into a C₃-C₄ epoxide.

2. A method as claimed in claim 1, **characterised in that** before converting the C₃OH and C₄OH are converted into a C₃-C₄ epoxide, the oxazoline compound is caused to react with an amount of a compound corresponding to formula IX 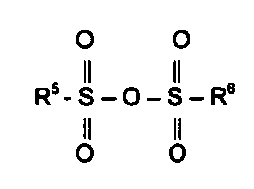 in which R⁵ and R⁶ may be the same or different and are as defined above, to convert the C₄OH group to sulfonyl protected group.

3. A method as claimed in claim 2, **characterised in that** the epoxide is contacted with an electrophilic reactant, followed by contacting it with a nucleophilic reactant to open the epoxide and cause in this product the elimination reaction to take place by the addition of an organic base to form the C₄-C₅ double bond.

4. A method as claimed in claim 3, **characterised in that** the compound comprising the C₄-C₅ double bond, is subjected to a washing procedure in weak acid medium so as to open the oxazoline, followed by a washing procedure in strong alkaline medium to remove the NH₂ protecting group.

## Patentansprüche

1. Verfahren zur Herstellung einer Sphingoidbase der Formel 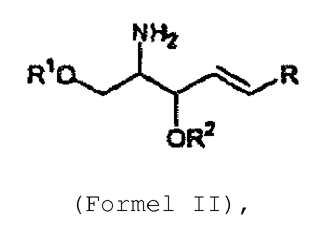 in der
R eine Kohlenwasserstoffkette mit 5-50, vorzugsweise 13-19, Kohlenstoffatomen bedeutet, bei der es sich um eine gerade oder verzweigte Kette handeln kann, die gesättigt sein kann oder eine oder mehrere Doppelbindungen enthalten kann, die eine oder mehrere funktionelle Gruppen enthalten kann,
R¹ und R² H bedeuten,
wobei das Verfahren die folgenden Schritte umfasst:
(1) Lösen einer Ausgangsverbindung der Formel (III) oder eines Salzes davon in einem im Wesentlichen inerten Lösungsmittel 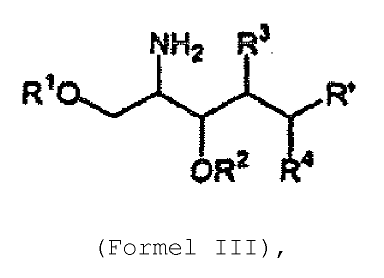 in der R¹, R² wie oben definiert sind,
R³ und R⁴ gleich oder verschieden sein können und H, OH oder eine Kohlenwasserstoffkette mit 1-10, vorzugsweise 1-5, Kohlenstoffatomen, die eine gerade oder verzweigte Kette sein kann, die gesättigt sein kann oder eine oder mehrere Doppelbindungen enthalten kann, die eine oder mehrere funktionelle Gruppen enthalten kann, bedeuten können,
und R' eine Kohlenwasserstoffkette mit 3-48, vorzugsweise 11-17, Kohlenstoffatomen die eine gerade oder verzweigte Kette sein kann, die gesättigt sein kann oder eine oder mehrere Doppelbindungen enthalten kann, die eine oder mehrere funktionelle Gruppen enthalten kann, wobei die funktionellen Gruppen vorzugsweise aus der Gruppe Hydroxylgruppe, Alkoxygruppe, primäres, sekundäres oder tertiäres Amin, Thiolgruppe, Thioethergruppe oder funktionelle phosphorhaltige Gruppe stammen, sein kann,
wobei als Verbindung der Formel III eine Verbindung entsprechend Formel V: 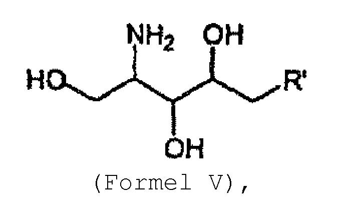 in der R' wie oben definiert ist, eingesetzt wird,
(2) Schützen der NH₂-Gruppe mit einer NH₂-Schutzgruppe durch Umsetzen der Verbindung der Formel III mit einer organischen Säure, einem Aktivester, einem Säurehalogenid, einem Säureanhydrid, zur Umwandlung der Aminfunktion in das entsprechende Amid,
(3) Aktivieren von C₄HR³ für eine Eliminierungsreaktion mit C₅HR⁴,
(4) Durchführen einer Eliminierungsreaktion unter Bildung einer Doppelbindung zwischen dem C₄- und dem C₅-Kohlenstoffatom,
(5) Entfernen der NH₂-Schutzgruppe,
**dadurch gekennzeichnet, dass** bei dem in Schritt (2) erhaltenen Amid die NH-Gruppe mit der C₁OH-Gruppe unter Bildung der entsprechenden Oxazolinverbindung umgesetzt wird und dass das C₃OH und das C₄OH in ein C₃-C₄-Epoxid umgewandelt werden.

2. Verfahren nach Anspruch 1, **dadurch gekennzeichnet, dass**, bevor das C₃OH und das C₄OH in ein C₃-C₄-Epoxid umgewandelt werden, die Oxazolinverbindung mit einer Menge einer Verbindung entsprechend Formel IX 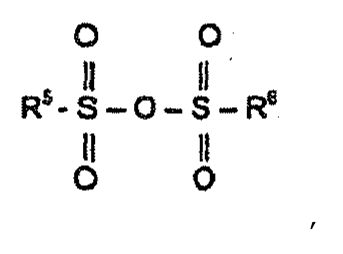 in der R⁵ und R⁶ gleich oder verschieden sein können und wie oben definiert sind, umgesetzt wird, um die C₄OH-Gruppe in eine sulfonylgeschützte Gruppe umzuwandeln.

3. Verfahren nach Anspruch 2, **dadurch gekennzeichnet, dass** das Epoxid mit einem elektrophilen Reaktanten in Kontakt gebracht wird, wonach es mit einem nukleophilen Reaktanten in Kontakt gebracht wird, wodurch das Epoxid geöffnet wird und die Eliminierungsreaktion in diesem Produkt durch Addition einer organischen Base unter Bildung der C₄-C₅-Doppelbindung stattfinden gelassen wird.

4. Verfahren nach Anspruch 3, **dadurch gekennzeichnet, dass** die Verbindung mit der C₄-C₅-Doppelbindung in einem schwach sauren Medium gewaschen wird, um das Oxazolin zu öffnen, wonach in einem stark alkalischen Medium gewaschen wird, um die NH₂-Schutzgruppe zu entfernen.

## Revendications

1. Procédé pour la préparation d'une base sphingoïde selon la formule 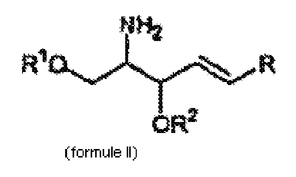 où
R représente une chaîne hydrocarbonée comprenant 5- 50, de préférence 13-19 atomes de carbone, qui peut être une chaîne linéaire ou ramifiée, qui peut être saturée ou contenir une ou plusieurs doubles liaisons, qui peut contenir un ou plusieurs groupes fonctionnels,
R¹ et R² représentent H,
ce procédé comprenant les étapes
(1) on dissout un composé de départ selon la formule III ou un sel de celui-ci dans un solvant substantiellement inerte, 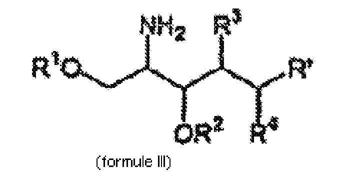 où R¹, R², sont définis comme ci-dessus
R³ et R⁴ peuvent être identiques ou différents et peuvent représenter H, OH ou une chaîne hydrocarbonée comprenant 1-10, de préférence 1-5 atomes de carbone, qui peut être une chaîne linéaire ou ramifiée, qui peut être saturée ou contenir une ou plusieurs doubles liaisons, qui peut contenir un ou plusieurs groupes fonctionnels, et
R' représente une chaîne hydrocarbonée comprenant 3-48, de préférence 11-17 atomes de carbone, qui peut être une chaîne linéaire ou ramifiée, qui peut être saturée ou contenir une ou plusieurs doubles liaisons, qui peut contenir un ou plusieurs groupes fonctionnels, les groupes fonctionnels étant de préférence choisis dans le groupe constitué par un groupe hydroxyle, un groupe alcoxy, une amine primaire, secondaire ou tertiaire, un groupe thiol, un thioéther ou un groupe fonctionnel contenant du phosphore,
où on utilise comme composé de formule III un composé correspondant à la formule V : 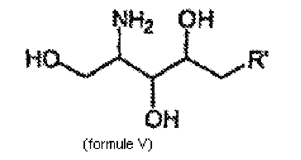 où R' est défini comme ci-dessus
(2) on protège le groupe NH₂ avec un groupe de protection de la fonction NH₂ en provoquant la réaction du composé de formule III avec un acide organique, un ester activé, un halogénure d'acide, un anhydride d'acide, pour convertir la fonction amine en amide correspondant,
(3) on active C₄HR³ pour une réaction d'élimination avec C₅HR⁴,
(4) on provoque la réalisation d'une réaction d'élimination pour former une double liaison entre l'atome de carbone C₄ et l'atome de carbone C₅,
(5) on élimine le groupe de protection de la fonction
NH₂
**caractérisé en ce que**, dans l'amide obtenu dans l'étape (2), on provoque la réaction du groupe NH avec le groupe C₁OH pour former le composé oxazoline correspondant et **en ce que** C₃OH et C₄OH sont convertis en un époxyde C₃-C₄.

2. Procédé selon la revendication 1, **caractérisé en ce qu'**avant la conversion de C₃OH et de C₄OH en un époxyde C₃-C₄, on provoque la réaction du composé oxazoline avec une quantité d'un composé correspondant à la formule IX 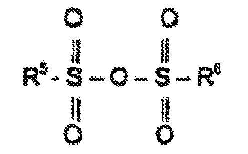 dans laquelle R⁵ et R⁶ peuvent être identiques ou différents et sont définis comme ci-dessus, pour convertir le groupe C₄OH en groupe sulfonyle protégé.

3. Procédé selon la revendication 2, **caractérisé en ce que** l'époxyde est mis en contact avec un réactif électrophile, puis on le met en contact avec un réactif nucléophile pour ouvrir l'époxyde et pour provoquer, dans ce produit, la réalisation de la réaction d'élimination par l'addition d'une base organique pour former la double liaison C₄-C₅.

4. Procédé selon la revendication 3, **caractérisé en ce que** le composé comprenant la double liaison C₄-C₅ est soumis à un procédé de lavage dans un milieu acide faible de manière à ouvrir l'oxazoline, suivi par un procédé de lavage dans un milieu alcalin fort pour éliminer le groupe de protection de la fonction NH₂.
